# EUROPÄISCHE PATENTSCHRIFT

(11) **EP 2 785 385 B1**
(45) Veröffentlichungstag und Bekanntmachung des Hinweises auf die Patenterteilung: **21.10.2015**
(21) Anmeldenummer: 12794936.0
(22) Anmeldetag: 27.11.2012
(51) Int. Cl.: A61L 9/20, A61L 9/014, D06F 25/00

(54) **DOMESTIC APPLIANCE WITH DEODORISING SYSTEM**
HAUSHALTSGERÄT MIT DESODORIERENDEM SYSTEM
APPAREIL MÉNAGER AVEC SYSTÈME DE DÉODORISATION

(30) Priorität: 01.12.2011 DE 102011087583
(43) Veröffentlichungstag der Anmeldung: 08.10.2014
(73) Patentinhaber: BSH Hausgeräte GmbH, 81739 München (DE)
(72) Erfinder: SCHAUB, Hartmut, 14656 Brieselang (DE); SCHULZE, Ingo, 16341 Panketal (DE); TILL, Christian, 10367 Berlin (DE)
(86) Internationale Anmeldenummer: PCT/EP2012/073673
(87) Internationale Veröffentlichungsnummer: WO 2013/079462

(56) Entgegenhaltungen:
- EP-A1- 1 602 774
- EP-A2- 1 602 766
- EP-A2- 2 213 947
- WO-A1-2010/139623
- WO-A2-2006/042740
- WO-A2-2006/065491
- WO-A2-2007/026387
- DE-U1- 20 021 301
- US-A1- 2003 113 246

## Beschreibung

Die Erfindung betrifft ein Haushaltsgerät mit einem Desodorierungssystem zum Entfernen geruchsaktiver Moleküle sowie ein Verfahren zum Entfernen geruchsaktiver Moleküle aus einem Prozessmedium eines Haushaltsgeräts.

Haushaltsgeräte werden während ihres Gebrauchs auf unterschiedliche Weise verunreinigt. So fallen beispielsweise in einer Geschirrspülmaschine Essensreste und in einer Waschmaschine oder einem Trockner Verunreinigungen aus den zu reinigenden Wäschestücken an. Aber nicht nur wasserführende Haushaltsgeräte, sondern auch Haushaltsgeräte wie Mikrowellen oder Backöfen werden während ihrer Benutzung verunreinigt. In Abhängigkeit von der Art des Haushaltsgeräts tritt die Problematik von Verunreinigungen somit in flüssigen und/oder in gasförmigen Prozessmedien des betreffenden Haushaltsgeräts auf. Solche Verunreinigungen, welche im Wesentlichen aus organischen Verbindungen bestehen, umfassen in der Regel eine Vielzahl von unterschiedlichen geruchsaktiven Molekülen. Bei geruchsaktiven Molekülen handelt es sich generell um chemische Verbindungen, die grundsätzlich eine ausreichende Flüchtigkeit, eine gewisse Lipophilie sowie eine hinreichende Wasserlöslichkeit aufweisen, um die über dem olfaktorischen Epithel der menschlichen Nase liegende wässrige Mucosa passieren zu können. Das maximale Molekülgewicht beträgt bei Aromastoffen ungefähr 300 g/mol. Diese Grenze ist einerseits physikalisch bedingt, da größere Moleküle kaum oder nicht mehr flüchtig sind. Andererseits handelt es sich hierbei auch um eine biologische Grenze, da zu voluminöse Moleküle nicht mehr mit biologischen Geruchsrezeptoren wechselwirken können.

Zur Geruchsbeseitigung in Haushaltsgeräten sind einzelne Verfahren wie Lüften des Prozessraums bzw. Verdünnen des Prozessmediums, Absorption der geruchsaktiven Moleküle über Wasserdampf, Adsorption der geruchsaktiven Moleküle an Aktivkohle, beispielsweise Aktivkohlefiltermatten im Prozessluftstrom eines Wäschetrockners, oder Ozonolyse der geruchsaktiven Moleküle bekannt. Ein Geschirrspülgerät mit einer Ozonolyse-Desodorierungseinrichtungen ist aus der JP 2005118209 A bekannt.

Die EP 1602774 A1 offenbart eine Waschmaschine, die unerwünschten Geruch mit Hilfe einer Desodorisierungseinrichtung aus Wäsche entfernt. Hierzu weist die Desodorisierungseinrichtung einen mit einem Photokatalysator beschichteten Filter sowie eine UV-Lampe auf.

Die WO 2010/139623 A1 betrifft ein Haushaltsgerät mit wenigstens einem Bauteil, dessen Oberfläche organischen Verschmutzungen ausgesetzt werden muss. Die Oberfläche weist einen Photokatalysator auf, der durch eine Lichtstrahlungsquelle mit aktivierender elektromagnetischer Strahlung bestrahlbar ist.

Die EP 2213947 A2 offenbart ein Hausgerät mit einem Photokatalysator, einer Lichtquelle zum Aktivieren des Photokatalysators und einem katalytischen Pfad, der sich zwischen einem Start- und einem Endpunkt erstreckt. Das Haushaltsgerät ist derart ausgebildet, dass ein Fluid vom Startpunkt zum Endpunkt durch den katalytischen Pfad geführt wird.

Die EP 1602766 A2 offenbart eine Waschmaschine, die eine desodorierende Einheit aufweist, welche eine Desodorierung unabhängig von einem Waschvorgang durchführt, um Gerüche aus der in die Waschmaschine gelegten Wäsche zu entfernen.

Aus der US 2003/113246 A1 ist eine Desodorierungseinrichtung bekannt, welche einen Gehäusekörper, einen Luft-Strömungskanal, zwei Lampen-Einheiten zum Erzeugen von Ozon, drei imprägnierte Photokatalysator-Filter und einen imprägnierten Kohlenstoff-Filter aufweist.

Die WO 2006/065491 A2 offenbart eine Luftaufbereitungsanlage mit einem Filter- und Heizelement, einer Plasma-Vorrichtung und einem Photokatalysator mit UV-Licht, um einen Luftstrom, der durch die Luftaufbereitungsanlage geleitet wird, zu reinigen. Die Luftaufbereitungsanlage wird in zwei verschiedenen Betriebsarten betrieben.

Die WO 2006/042740 A2 offenbart ein Verfahren zum Sterilisieren von Luft in einem Luftkanal, sowie eine Verwendung einer Vorrichtung zum Abbau von gasförmigen Kohlenwasserstoff-Emissionen. Gemäß dem beanspruchten Verfahren wird Luft in einem Luftkanal mittels einer UV-Anlage mit UV-Strahlung beaufschlagt und die so vorgereinigte Luft mittels einer nachgeschalteten Ionisationseinheit im Luftkanal ionisiert.

Die DE 20021301 U1 offenbart einen Luftreiniger, welcher ein Hauptgehäuse, das einen Lufteinlass und einen Luftauslass enthält, und Aktivkohle und Filter umfasst, die bis an den Lufteinlass angeordnet sind, wobei die Luft vom Lufteinlass über die Aktivkohle und Filter mittels eines Ventilators abgeleitet und anschließend durch den Luftauslass wieder abgegeben wird. Das Filter ist aus übereinander geschichteten Kunststoffnetzlagen durch Spritzgießen hergestellt, wobei jede Netzlage eine Vielzahl von Trennrippen und eingravierten Öffnungen besitzt, so dass der Fotokatalysator auf den Trennrippen angeordnet werden kann und diese Struktur es ermöglicht, dass Luft durch die eingravierten Öffnungen in die Netzlagen strömen und eine fotokatalytische Reaktion stattfinden kann, die von der auf den auf den Trennrippen befindlichen Fotokatalysator auffallenden Ultraviolettstrahlung verursacht wird und die Luft zersetzen und desinfizieren kann.

Die WO 2007/026387 A2 offenbart Filter verschiedener Art für den Einsatz in Klimaanlagen und Reinigungssystemen, insbesondere Filter mit photokatalytischen Materialien und Nanomaterialien, welche die Dekontamination von bakteriellen und viralen Verunreinigungen auch in Abwesenheit von Licht-Strahlung erlauben.

Jedes dieser Verfahren weist neben spezifischen Vorteilen jedoch auch eine Reihe von Nachteilen auf. Das Lüften des Prozessraums bzw. das Verdünnen des Prozessmediums führt zu langen Prozesszeiten. Darüber hinaus besteht die Gefahr der Akkumulation von geruchsaktiven Molekülen in geschlossenen Prozesskreisen, so dass sich das Verfahren nur für Haushaltsgeräte mit offenen Prozessräumen eignet. Entsprechend werden die geruchsaktiven Moleküle bei der Absorption über Wasserdampf nicht zersetzt, sondern lediglich an einen anderen Ort transportiert, so dass auch hier die Gefahr einer Geruchsakkumulation in strömungsberuhigten Bereichen des Haushaltsgeräts besteht. Bei der Verwendung von Aktivkohle kommt es im Laufe der Zeit zwangsläufig zu einer Verunreinigung der Aktivkohleoberflächen und damit zu einer Verringerung der Säuberungsleistung. Nachteilig an allen adsorptiven und absorptiven Verfahren ist zudem, dass nicht alle geruchsaktiven Molekülklassen auf Adsorberflächen aufziehen bzw. dauerhaft am Absorber gebunden bleiben und dass generell lediglich die Lokalisation der Gerüche verändert, der Geruch selbst aber nicht dauerhaft beseitigt wird.

Reaktive Anwendungen, wie etwa die Nutzung von Ozon zur Reinigung des Prozessmediums, sind zwar in der Lage, Gerüche dauerhaft zu beseitigen, weisen jedoch den Nachteil auf, dass die Erzeugung und Verwendung von Ozon nicht oder nur schwer auf kleinere Reaktionsräume begrenzt werden kann. Daher sollte das Haushaltsgerät zur Vermeidung von Ozonaustritt in die Umgebung gegen Gasaustritt gesichert werden. Abhängig von der Ozonkonzentration und der Konzentration der vorhandenen Reaktionspartner kann die Abklingzeit jedoch einige Stunden betragen, was für Haushaltsgeräte in der Regel unerwünscht ist.

Aufgabe der vorliegenden Erfindung ist es, ein Haushaltsgerät zu schaffen, welches eine möglichst schnelle und vollständige Entfernung von geruchsaktiven Molekülen aus einem Prozessmedium des Haushaltsgeräts ermöglicht.

Die Aufgabe wird erfindungsgemäß durch ein Haushaltsgerät mit einem Desodorierungssystem zum Entfernen geruchsaktiver Moleküle aus einem Prozessmedium des Haushaltsgeräts gemäß dem unabhängigen Patentanspruch gelöst. Vorteilhafte Ausgestaltungen mit zweckmäßigen Weiterbildungen der Erfindung sind in abhängigen Patentansprüchen sowie nachfolgender Beschreibung und beigefügter Zeichnung angegeben.

Ein Haushaltsgerät, welches eine möglichst schnelle und vollständige Entfernung von geruchsaktiven Molekülen aus einem Prozessmedium des Haushaltsgeräts ermöglicht, ist dadurch geschaffen, dass das Haushaltsgerät ein Desodorierungssystem aufweist, welches in einem zumindest während des Entfernens der geruchsaktiven Moleküle verschlossenen Prozessraum des Haushaltsgeräts angeordnet ist und wenigstens drei unterschiedliche, mit dem Prozessmedium beaufschlagbare Desodorierungseinrichtungen umfasst. Mit anderen Worten ist es vorgesehen, dass das Haushaltsgerät ein Desodorierungssystem umfasst, welches ausgebildet ist, zur Geruchsbeseitigung eine Kombination von wenigstens zwei unterschiedlichen Desodorierungsverfahren in einem geschlossenen Prozessraum durchzuführen. Der Prozessraum des Haushaltsgeräts kann dabei beispielsweise ein in sich geschlossener Kreislauf für ein Prozessmedium sein. Weiterhin kann vorgesehen sein, dass der Prozessräum permanent oder nur während des Entfernens der geruchsaktiven Moleküle geschlossen ist. Das Desodorierungssystem ist bevorzugt ausgebildet, wenigstens zwei unterschiedliche Desodorierungsverfahren bzw. die diese Desodorierungsverfahren ausführenden Desodorierungseinrichtungen gleichzeitig oder getrennt voneinander zu aktivieren bzw. zu deaktivieren. Ein Prozessmedium im Sinne der vorliegenden Erfindung ist jedes flüssige oder feste Medium, das im betroffenen Haushaltsgerät verwendet wird oder anfällt. Beispielsweise ist unter einem Prozessmedium erwärmte Luft in einem Trockner oder Backofen, die Waschlauge einer Waschmaschine oder eines Geschirrspülgeräts, aber auch die Abluft einer Waschmaschine oder eines Geschirrspülgeräts zu verstehen. Durch die Kombination unterschiedlicher Desodorierungseinrichtungen ergeben sich in Verbindung mit dem geschlossenen Prozessraum verschiedene quantitative und qualitative Vorteile. Beispielsweise kann zur Vermeidung langer Abklingzeiten zusätzlich zu einer ozonolytischen Desodorierungseinrichtung eine photokatalytische Desodorierungseinrichtung vorgesehen sein. Die Nutzung einer photokatalytischen Desodorierungseinrichtung bietet den Vorteil sehr kurzer Halbwertszeiten der erzeugten Radikale, insbesondere von aktiviertem Sauerstoff, so dass die Abklingzeiten im Vergleich zu einer Ozonolyse wesentlich verringert werden und das Haushaltsgerät schneller wieder freigegeben werden kann. Zusätzlich können diese Abklingzeiten auch durch die Kombination einer ozonolytischen Desodorierungseinrichtung mit einer Desodorierungseinrichtung, welche einen geeigneten Reaktionspartner bereitstellt, verringert werden. Ein geeigneter Reaktionspartner kann dabei beispielsweise eine einen Aktivkohlefilter umfassende Filtrations- oder Adsorptionseinrichtungen sein, die in Verbindung mit einer ozonolytischen Desodorierungseinrichtung synergistisch sowohl zur Adsorption von geruchsaktiven Molekülen als auch zur Reaktion mit Ozon verwendet werden kann. Alternativ oder zusätzlich können nicht mit Ozon reagierende Filtrationseinrichtungen vorgesehen sein, wobei auf diesen Filtrationseinrichtungen abgelagerte geruchsaktive Moleküle als Reaktionspartner für Ozon oder aktivierten Sauerstoff dienen und abgebaut werden. Dabei können gleichzeitig durch Ablagerungen verschlossene Öffnungen und Durchgänge im Filtermaterial durch die Reaktion mit Ozon wieder "freigebrannt" werden. Die Filtrationseinrichtung wird dementsprechend mit Hilfe der ozonolytischen Desodorierungseinrichtung regeneriert, wodurch ihre ursprüngliche Filtrationswirkung wesentlich länger erhalten bleibt. Weiterhin kann vorgesehen sein, dass vernebeltes und/oder verdampftes Wasser in einer Absorptionseinrichtung für die Absorption der geruchsaktiven Moleküle und/oder als Transportmedium zu einer die geruchsaktiven Moleküle degradierenden Desodorierungseinrichtung verwendet wird. Des Weiteren können die geruchsaktiven Moleküle mit Hilfe einer degradierenden Desodorierungseinrichtung - insbesondere einer thermischen, katalytischen, oxidativen und/oder biologischen Desodorierungseinrichtung - zersetzt werden. Hierbei werden grundsätzlich aus langkettigen und schwerer in Wasser löslichen Kohlenwasserstoffverbindungen kurzkettigere und leichter lösliche Moleküle gebildet, die dementsprechend leichter von einer stromab angeordneten bzw. nachgeschalteten Ad- oder Absorptionseinrichtungen des Desodorierungssystems aufgenommen werden können. Zusammenfassend kann das erfindungsgemäße Haushaltsgerät somit besonders einfach an eine große Vielzahl möglicher geruchsaktiver Moleküle angepasst werden. Darüber hinaus kann das Desodorierungssystem besonders einfach und flexibel an die betreffende Ausgestaltung des Haushaltsgeräts angepasst werden. Weiterhin können die einzelnen, durch die wenigstens zwei unterschiedlichen Desodorierungseinrichtungen durchführbaren Desodorierungsschritte besser gesteuert werden. Zudem ist eine Selbstreinigung etwaiger Filterelemente wie beispielsweise Aktivkohle- oder Membranfilter durch Reaktion der abgetrennten geruchsaktiven Moleküle mit Ozon ermöglicht. Vorzugsweise ist das Desodorierungssystem ausgebildet, im Mittel wenigstens 60 Gew.-%, vorzugsweise wenigstens 80 Gew.-% und besonders bevorzugt wenigstens 90 Gew.-% der im Prozessmedium enthaltenen aromaaktiven Moleküle aus dem Prozessmedium zu entfernen.

Weitere Vorteile ergeben sich, wenn das Haushaltsgerät als Kühlschrank, Herd, Mikrowellengerät, als wasserführendes Haushaltsgerät, insbesondere als Geschirrspülgerät, Wäschebehandlungsgerät, bevorzugt aus der Gruppe Waschmaschine, Trockner und Waschtrockner, ausgebildet ist. Das Haushaltsgerät ist bevorzugt ein Wäschebehandlungsgerät aus der Gruppe Waschmaschine, Trockner und Waschtrockner. Im Allgemeinen weist ein Wäschebehandlungsgerät eine drehbar gelagerte Trommel, einen Antriebsmotor für die Trommel und eine Heizeinrichtung auf. Zudem sind in der Regel Schaltmittel und Antriebsmittel zum Drehen und Anhalten der Trommel vorhanden. Ein Trockner umfasst im Allgemeinen einen Prozessluftkanal, in dem sich eine Trocknungskammer für die zu trocknenden Gegenstände und im Allgemeinen eine Heizung zur Erwärmung der Prozessluft und ein Gebläse zur Beförderung der Prozessluft befinden. In einem Trockner wird zum Trocknen von Gut wie Wäsche Prozessluft durch einen Prozessraum mit dem zu trocknenden Gut geführt. Aus der Trocknungskammer strömende feuchtwarme Prozessluft wird zum Auskondensieren der von dem feuchten Gut herrührenden Feuchte in einem geeigneten Wärmetauscher (Luft-Luft-Wärmetauscher, Wärmesenke einer Wärmepumpe) abgekühlt und nach Abtrennen des entstandenen Kondensats wieder mittels einer Heizung (elektrische Heizung, Gasheizung oder Wärmequelle einer Wärmepumpe) erhitzt und in die Trocknungskammer zurück geleitet. In einem Ablufttrockner sind dagegen ein Zuluftkanal und ein Abluftkanal vorhanden, so dass die aus einer Umgebung des Ablufttrockners (insbesondere einem Aufstellraum) in den Zuluftkanal gesaugte Prozessluft nach Durchgang durch die Trocknungskammer über den Abluftkanal zu einem Abluftausgang und damit wieder in den Aufstellraum, oder durch eine entsprechende Abluftleitung aus diesem heraus, geführt wird. Eine Waschmaschine umfasst im Allgemeinen neben einer Trommel als Aufnahmebehälter für die zu behandelnden Wäschestücke einen Laugenbehälter, ein Wasserzulaufsystem und ein am Boden des Laugenbehälters angeordnetes Laugenablaufsystem mit einer Laugenpumpe. Ein Waschtrockner vereinigt die Merkmale eines Trockners und einer Waschmaschine in sich und ist für beide entsprechenden Verwendungen nutzbar. Das erfindungsgemäße Haushaltsgerät ist jedoch hinsichtlich seiner bestimmungsgemäßen Verwendung grundsätzlich nicht eingeschränkt.

Erfindungsgemäß umfasst das Desodorierungssystem wenigstens drei Desodorierungseinrichtungen aus der Gruppe der Filtrationseinrichtungen, Extraktionseinrichtungen, und oxidativen Desodorierungseinrichtungen. Hierdurch kann zumindest die weit überwiegende Anzahl an geruchsaktiven Molekülen durch die Kombination von wenigstens drei Desodorierungseinrichtungen zumindest im Wesentlichen aus dem Prozessmedium des Haushaltsgeräts entfernt werden, wodurch eine besonders schnelle und umfassende Geruchsentfernung ermöglicht ist. Dabei kann in Abhängigkeit des jeweiligen Haushaltsgeräts vorgesehen sein, dass vier, fünf oder sechs unterschiedliche Desodorierungseinrichtungen vorgesehen sind. Alternativ oder zusätzlich kann vorgesehen sein, dass zwei oder mehr Desodorierungseinrichtungen des gleichen Typs parallel und/oder nacheinander im Prozessmediumstrom angeordnet sind.

Weitere Vorteile ergeben sich, indem dem Desodorierungssystem eine Verschließeinrichtung zugeordnet ist, welche zwischen einer den Prozessraum des Haushaltsgeräts verschließenden Schließstellung und einer den Prozessraum des Haushaltsgeräts freigebenden Offenstellung schaltbar ist. Hierdurch kann der Prozessraum, in welchem sich das von geruchsaktiven Molekülen zu reinigende Prozessmedium befindet, zumindest für die Dauer der Desodorisierung bzw. bei aktiviertem Desodorierungssystem mit Hilfe der Verschließeinrichtung geschlossen und anschließend bzw. bei deaktiviertem Desodorierungssystem wieder freigegeben werden. Die Verschließeinrichtung kann hierzu beispielsweise eine Klappe umfassen, mittels welcher ein Strömungsweg des Prozessmediums verschließbar und öffenbar ist.

In einer weiteren vorteilhaften Ausgestaltung der Erfindung ist vorgesehen, dass das Desodorierungssystem zum Abbau von geruchsaktiven Molekülen in einem flüssigen und/oder gasförmigen Prozessmedium ausgebildet ist. Dies ermöglicht eine besonders gute Anpassung des Desodorierungssystems und damit der erzielbaren Reinigungswirkung an die jeweilige Ausgestaltung des Haushaltsgeräts.

In einer weiteren vorteilhaften Ausgestaltung der Erfindung ist vorgesehen, dass das Desodorierungssystem ausgebildet ist, die wenigstens zwei Desodorierungseinrichtungen gleichzeitig und/oder nacheinander mit dem Prozessmedium zu beaufschlagen. Hierdurch kann der Reinigungsprozess optimal an die Art und Konzentration der zu entfernenden geruchsaktiven Moleküle angepasst werden.

In einer vorteilhaften Ausgestaltung der Erfindung ist vorgesehen, dass der Prozessraum des Haushaltsgeräts zumindest während dem Entfernen der geruchsaktiven Moleküle mittels einer Verschließeinrichtung verschlossen und/oder nach dem Entfernen der geruchsaktiven Moleküle mittels der Verschließeinrichtung geöffnet wird. Durch das Schließen des Prozessraums ist sichergestellt, dass während des Desodorisierens keine geruchsaktiven Moleküle in die Umgebung austreten, so dass es zu keiner Geruchsbelästigung durch das Haushaltsgerät kommt. Weiterhin wird hierdurch eine zumindest weitgehende Entfernung der geruchsaktiven Moleküle aus dem Prozessmedium erleichtert, da kein Stoffaustausch mit der Umgebung möglich ist. Beispielsweise wird bei einem als Trockner ausgebildeten Haushaltsgerät die mit geruchsaktiven Molekülen beladene Prozessluft während dem Entfernen in einem geschlossenen Kreislauf durch eine Trocknungskammer mit dem zu trocknenden Gut zu einem Desodorierungssystem geleitet, mittels welchem die geruchsaktiven Moleküle ganz oder teilweise aus der Prozessluft entfernt werden. Anschließend wird die desodorisierte Prozessluft wieder in die Trocknungskammer zurückgeführt.

In einer weiteren vorteilhaften Ausgestaltung der Erfindung ist vorgesehen, dass geruchsaktive Moleküle im Prozessmedium mittels des Desodorierungssystems mittels Wasserdampf, welcher durch Verdampfen und/oder Vernebeln erzeugt wird, zu einer Filtereinrichtung, insbesondere einem Aktivkohlefilter, transportiert, am Filtermaterial der Filtereinrichtung abgelagert und photolytisch und/oder ozonolytisch degradiert werden. Auf diese Weise wird eine besonders effiziente, schnelle und zumindest weitgehend vollständige Entfernung der geruchsaktiven Moleküle aus dem Prozessmedium erreicht, wobei die einzelnen Verfahrensschritte synergistisch zusammenwirken. So fungiert der Nebel oder Dampf nicht nur als Absorber für die Geruchsmoleküle, sondern transportiert diese zusätzlich zur Filtereinrichtung. Eine als Aktivkohlefilter ausgebildete Filtereinrichtung reduziert dabei nicht nur die Konzentration der geruchsaktiven Moleküle im Prozessmedium, sondern fungiert zusätzlich als Reaktionspartner für Ozon. Alternativ oder zusätzlich können auch nicht mit Ozon reagierende Filter verwendet werden, wobei die an diesen abgeschiedenen Kohlenwasserstoffe als Reaktionspartner für Ozon oder andere reaktive Sauerstoffspezies dienen. Dabei werden gegebenenfalls durch die Ablagerungen verschlossene Öffnungen im Filter durch die Reaktion mit Ozon wieder "freigebrannt". Des Weiteren werden bei der photolytischen und/oder ozonolytischen Reaktion aus längerkettigen, schwer löslichen Kohlenwasserstoffen kürzerkettige und damit leichter lösliche Moleküle gebildet, die entsprechend einfacher von einem Ad- oder Absorber aus dem Prozessmedium entfernt werden können. Schließlich ermöglicht die Kombination aus ozonolytischer und/oder photolytischer Degradierung und einem geeigneten Filter eine erhebliche Verringerung der Abklingzeiten, so dass der Prozessraum des Haushaltsgeräts entsprechend schnell wieder freigegeben werden kann. Es können grundsätzlich allerdings auch andere additive oder alternative Verfahrensschritte vorgesehen sein.

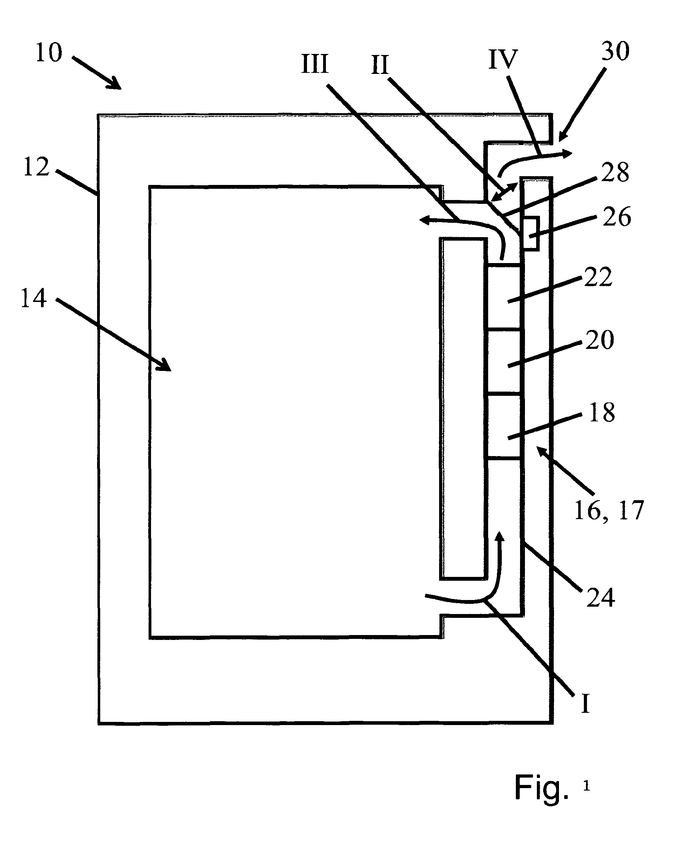

Weitere Merkmale der Erfindung ergeben sich aus den Patentansprüchen, dem Ausführungsbeispiel sowie anhand der Zeichnung.

Ein bevorzugtes Ausführungsbeispiel wird nachfolgend anhand seiner Darstellung in der beigefügten Zeichnung näher erläutert Dabei zeigt die einzige Figur der Zeichnung schematisch einen vertikalen Schnitt durch ein als Trockner ausgebildetes Haushaltsgerät.

Die einzige Figur zeigt schematisch einen vertikalen Schnitt durch ein als Trockner ausgebildetes Haushaltsgerät 10. Das Haushaltsgerät 10 umfasst ein Gehäuse 12, in welchem ein Prozessraum 14 zum Trocknen von Wäsche oder anderem Trockengut vorgesehen ist. Der Prozessraum 14 kann beispielsweise eine drehbar gelagerte Trommel (nicht gezeigt) aufweisen, welcher in an sich bekannter und daher nicht näher gezeigter Weise ein Antriebsmotor für die Trommel und eine Heizeinrichtung zugeordnet sind. Zudem kann das Haushaltsgerät 10 in an sich bekannter Weise Steuergeräte, Schaltmittel und Antriebsmittel zum Drehen und Anhalten der Trommel aufweisen. In dem Haushaltsgerät 10 wird zum Trocknen von Gut wie Wäsche Prozessluft als Prozessmedium durch den Prozessraum 14 mit dem zu trocknenden Gut geführt. Zum Entfernen geruchsaktiver Moleküle aus dem Prozessmedium des Haushaltsgeräts 10 weist dieses ein Desodorierungssystem 16 auf, welches seinerseits drei unterschiedliche Desodorierungseinrichtungen 17, nämlich vorliegend eine Extraktionseinrichtung 18, eine Filtrationseinrichtung 20 sowie eine oxidative Desodorierungseinrichtung 22, umfasst. Das Desodorierungssystem 16 ist in einem fluidisch mit dem Prozessraum 14 gekoppelten Kanal 24 angeordnet, durch welchen die Prozessluft gemäß Pfeil I geleitet wird. Stromab des Desodorierungssystems 16 ist eine Verschließeinrichtung 26 mit einer Klappe 28 angeordnet. Die Klappe 28 kann gemäß Doppelpfeil II zwischen der gezeigten, den Prozessraum 14 verschließenden Schließstellung und einer den Prozessraum 14 des Haushaltsgeräts 10 freigebenden Offenstellung bewegt werden. Vorzugsweise erfolgt die Steuerung des Desodorierungssystems 16 und der Verschließeinrichtung 26 mit Hilfe eines zentralen Steuergeräts (nicht gezeigt) des Haushaltsgeräts 10. Bei geöffneter Klappe 28 kann die Prozessluft durch einen Abluftkanal 30 aus dem Prozessraum 14 und aus dem Haushaltsgerät 10 ausgeleitet werden. Solange das Desodorierungssystem 16 aktiviert ist, wird die Klappe 28 in die Schließstellung bewegt, so dass die Prozessluft in einem geschlossenen Kreisprozess gemäß Pfeil III zurück in den Prozessraum 14 geleitet wird. Nach Beendigung des Geruchsentfernungsverfahrens kann die Klappe 28 geöffnet und der Prozessraum 14 gemäß Pfeil IV entlüftet werden.

Im gezeigten Ausführungsbeispiel ist die Extraktionseinrichtung 18 als Wasserverneblungseinrichtung ausgebildet, mittels welcher ein kalter Wassernebel erzeugt wird. Der Wassernebel extrahiert geruchsaktive Moleküle aus der Prozessluft und transportiert die geruchsaktiven Moleküle weiter zu der als Aktivkohlefilter ausgebildeten Filtrationseinrichtung 20, in welcher die Prozessluft gefiltert und geruchsaktiven Moleküle adsorbiert werden. Die gezeigte Filtrationseinrichtung 20 fungiert somit gleichzeitig als Filter und als Adsorber. Die oxidative Desodorierungseinrichtung 22 ist ihrerseits als Ozonolyseeinrichtungen, bei welchen durch Koronaentladung eine ozonhaltige Luftströmung erzeugt wird, ausgebildet. Durch die Kombination mit der als Aktivkohlefilter ausgebildeten Filtrationseinrichtung 20 wird den reaktiven Sauerstoffspezies zudem ein geeigneter Reaktionspartner zur Verfügung gestellt, wodurch die Abklingzeiten des Verfahrens weiter reduziert werden. Der Aktivkohlefilter erfüllt somit eine Doppelfunktion, indem er einerseits Geruchsmoleküle absorbiert und andererseits zur Reaktion mit aktiviertem Sauerstoff genutzt wird. Dabei werden gegebenenfalls durch Ablagerungen verschlossene Öffnungen in der Filtrationseinrichtung 20 durch die Reaktion mit dem aktivierten Sauerstoff wieder "freigebrannt" und die Filtrationseinrichtung 20 somit regeneriert. Es kann vorgesehen sein, dass die Filtrationseinrichtung 20 und die oxidative Desodorierungseinrichtung 22 parallel oder in Strömungsrichtung nacheinander im Kanal 24 angeordnet sind und dementsprechend gleichzeitig oder nacheinander auf die Prozessluft einwirken.

Das Haushaltsgerät 10 bietet somit verschiedene Vorteile. Ein wesentlicher Vorteil besteht darin, dass eine große Vielzahl an möglicherweise auftretenden Gerüchen durch die Kombination der unterschiedlichen Geruchsentfernungs-Teilverfahren gezielt und einfach steuerbar aus dem Prozessmedium entfernt werden kann. Des Weiteren wird eine vorteilhafte Selbstreinigung des beteiligten Aktivkohle- oder auch eines Membranfilters durch Reaktion der abgesonderten Schichten mit Ozon oder aktiviertem Sauerstoff erzielt.

### Bezugszeichenliste

- 10: Haushaltsgerät
- 12: Gehäuse
- 14: Prozessraum
- 16: Desodorierungssystem
- 17: Desodorierungseinrichtung
- 18: Extraktionseinrichtung
- 20: Filtrationseinrichtung
- 22: oxidative Desodorierungseinrichtung
- 24: Kanal
- 26: Verschließeinrichtung
- 28: Klappe
- 30: Abluftkanal
- I: Pfeil
- II: Doppelpfeil
- III: Pfeil
- IV: Pfeil

## Patentansprüche

1. Haushaltsgerät (10) mit einem Desodorierungssystem (16) zum Entfernen geruchsaktiver Moleküle aus einem Prozessmedium des Haushaltsgeräts (10), wobei das Desodorierungssystem (16) in einem zumindest während des Entfernens der geruchsaktiven Moleküle geschlossenen Prozessraum (14) des Haushaltsgeräts (10) angeordnet ist und wenigstens drei unterschiedliche, mit dem Prozessmedium beaufschlagbare Desodorierungseinrichtungen (17) umfasst, wobei das Desodorierungssystem (16) in einem fluidisch mit dem Prozessraum (14) gekoppelten Kanal (24) angeordnet ist, durch welchen die Prozessluft geleitet wird, und wenigstens drei Desodorierungseinrichtungen (17) aus der Gruppe der Filtrationseinrichtungen (20), Extraktionseinrichtungen (18) und oxidativen Desodorierungseinrichtungen (22) umfasst, dass die Extraktionseinrichtungen (18) als Wasserverneblungseinrichtung ausgebildet ist, mittels welcher ein kalter Wassernebel erzeugt wird, dass die Filtrationseinrichtungen (20) einen Aktivkohlefilter aufweist, welcher die Prozessluft filtert und geruchsaktive Moleküle adsorbiert, und dass die oxidativen Desodorierungseinrichtungen (22) eine Ozonolyseeinrichtung umfasst.

2. Haushaltsgerät (10) nach Anspruch 1, **dadurch gekennzeichnet, dass** dieses als Kühlschrank, Herd, Mikrowellengerät, als wasserführendes Haushaltsgerät, insbesondere als Geschirrspülgerät, Wäschebehandlungsgerät, bevorzugt aus der Gruppe Waschmaschine, Trockner und Waschtrockner, ausgebildet ist.

3. Haushaltsgerät (10) nach einem der Ansprüche 1 oder 2, **dadurch gekennzeichnet, dass** dem Desodorierungssystem (16) eine Verschließeinrichtung (26) zugeordnet ist, welche zwischen einer den Prozessraum (14) des Haushaltsgeräts (10) verschließenden Schließstellung und einer den Prozessraum (14) des Haushaltsgeräts (10) freigebenden Offenstellung schaltbar ist.

4. Haushaltsgerät (10) nach einem der Ansprüche 1 bis 3, **dadurch gekennzeichnet, dass** das Desodorierungssystem (16) zum Abbau von geruchsaktiven Molekülen in einem flüssigen und/oder gasförmigen Prozessmedium ausgebildet ist.

5. Haushaltsgerät (10) nach einem der Ansprüche 1 bis 4, **dadurch gekennzeichnet, dass** das Desodorierungssystem (16) ausgebildet ist, die wenigstens zwei Desodorierungseinrichtungen (17) nacheinander mit dem Prozessmedium zu beaufschlagen.

## Claims

1. Domestic appliance (10) having a deodorising system (16) for removing odour-producing molecules from a process medium of the domestic appliance (10), wherein the deodorising system (16) is arranged in a processing room (14) of the domestic appliance (10) which is closed at least during removal of the odour-producing molecules and comprises at least three different deodorising facilities (17) to which the process medium can be applied, wherein the deodorising system (16) is arranged in a channel (24) coupled fluidically to the processing room (14), through which the process air is conducted, and comprises at least three deodorising facilities (17) from the group comprising filtration facilities (20), extraction facilities (18) and oxidative deodorising facilities (22), such that the extraction facilities (18) are embodied as a water atomisation facility, by means of which a cold fog is produced, that the filtration facilities (20) have an active carbon filter, which filters the process air and absorbs odour-producing molecules and that the oxidative deodorising facilities (22) comprise an ozonolysis facility.

2. Domestic appliance (10) according to claim 1, **characterised in that** this is embodied as a refrigerator, cooker, microwave, as a water-conducting domestic appliance, in particular as a dishwasher, laundry treatment device, preferably from the group comprising washing machine, dryer and washer-dryer.

3. Domestic appliance (10) according to one of claims 1 or 2, **characterised in that** a closing facility (26) is assigned to the deodorising system (16), which can be switched between a closing position closing the processing room (14) of the domestic appliance (10) and an open position opening the processing room (14) of the domestic appliance (10).

4. Domestic appliance (10) according to one of claims 1 to 3, **characterised in that** the deodorising system (16) is embodied to break down odour-producing molecules in a liquid and/or gaseous process medium.

5. Domestic appliance (10) according to one of claims 1 to 4, **characterised in that** the deodorising system (16) is embodied to apply the at least two deodorising facilities (17) successively with the process medium.

## Revendications

1. Appareil ménager (10) avec un système de désodorisation (16) pour éliminer les molécules odorantes d'un fluide de processus de l'appareil ménager (10), le système de désodorisation (16) étant disposé dans un espace de processus (14) de l'appareil électroménager (10), fermé au moins pendant l'élimination des molécules odorantes, et comprenant au moins trois dispositifs de désodorisation (17) différents, alimentés par le fluide de processus, le système de désodorisation (16) étant disposé dans un canal (24) en liaison fluidique avec l'espace de processus (14) et à travers lequel passe l'air de processus, et comprenant au moins trois dispositifs de désodorisation (17) issus du groupe des dispositifs de filtration (20), des dispositifs d'extraction (18) et des dispositifs de désodorisation par oxydation (22), les dispositifs d'extraction (18) étant réalisés sous forme de dispositif nébuliseur d'eau permettant de produire un brouillard d'eau froide, les dispositifs de filtration (20) comportant un filtre à charbon actif qui filtre l'air de processus et adsorbe les molécules odorantes, et les dispositifs de désodorisation par oxydation (22) comprenant un dispositif d'ozonolyse.

2. Appareil ménager (10) selon la revendication 1, **caractérisé en ce qu'**il est réalisé sous forme de réfrigérateur, de four, d'appareil à micro-ondes, sous forme d'appareil ménager à circulation d'eau, notamment sous forme de lave-vaisselle, d'appareil de traitement du linge, de préférence issu du groupe comprenant le lave-linge, le sèche-linge et le lave-linge séchant.

3. Appareil ménager (10) selon l'une des revendications 1 ou 2, **caractérisé en ce qu'**un dispositif de fermeture (26) est associé au système de désodorisation (16), lequel dispositif est commutable entre une position de fermeture fermant l'espace de processus (14) de l'appareil ménager (10) et une position d'ouverture libérant l'espace de processus (14) de l'appareil ménager (10).

4. Appareil ménager (10) selon l'une des revendications 1 à 3, **caractérisé en ce que** le système de désodorisation (16) est conçu pour éliminer les molécules odorantes dans un fluide de processus liquide et/ou gazeux.

5. Appareil ménager (10) selon l'une des revendications 1 à 4, **caractérisé en ce que** le système de désodorisation (16) est conçu pour alimenter les au moins deux dispositifs de désodorisation (17) l'un après l'autre avec le fluide de processus.
